(19) Europäisches Patentamt
European Patent Office
Office européen des brevets

(11) EP 3 808 353 B1

(12) EUROPEAN PATENT SPECIFICATION

(45) Date of publication and mention of the grant of the patent:
**16.08.2023 Bulletin 2023/33**

(21) Application number: **20199450.6**

(22) Date of filing: **30.09.2020**

(51) International Patent Classification (IPC):
***A61K 31/715*** (2006.01) ***A61K 31/728*** (2006.01)
***A61P 27/04*** (2006.01) ***A61P 27/02*** (2006.01)
***A61K 45/06*** (2006.01)

(52) Cooperative Patent Classification (CPC):
(C-Sets available)
**A61P 27/02; A61K 31/715; A61K 31/728;**
**A61P 27/04** (Cont.)

# (54) COMPOSITION AND USE OF AN OPHTHALMIC SOLUTION BASED ON HYALURONIC ACID AND ARABINOGALACTAN

ZUSAMMENSETZUNG UND VERWENDUNG EINER OPHTHALMISCHEN LÖSUNG AUF DER BASIS VON HYALURONSÄURE UND ARABINOGALAKTAN

COMPOSITION ET UTILISATION D'UNE SOLUTION OPHTALMIQUE À BASE D'ACIDE HYALURONIQUE ET D'ARABINOGALACTAN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2019 IT 201900018929**

(43) Date of publication of application:
**21.04.2021 Bulletin 2021/16**

(73) Proprietor: **MD Italy Srl**
**00041 Albano Laziale (RM) (IT)**

(72) Inventors:
- **De Grazia, Domenico**
  **00124 Rome (RM) (IT)**
- **De Grazia, Giulia**
  **00124 Rome (RM) (IT)**
- **Farinelli, Francesco**
  **05022 Amelia (TR) (IT)**
- **Remiddi, Stefano**
  **00142 Rome (RM) (IT)**
- **Silvani, Ludovica**
  **00137 Rome (RM) (IT)**

(74) Representative: **Sarpi, Maurizio et al**
**Studio Ferrario S.r.l.**
**Via Collina, 36**
**00187 Roma (IT)**

(56) References cited:
**WO-A1-2009/018060 WO-A2-2007/020671**

- **ANUBHA KHARE ET AL: "Mucoadhesive Polymers for Enhancing Retention in Ocular Drug Delivery: A Critical Review", REVIEWS OF ADHESION AND ADHESIVES, vol. 2, no. 4, 1 November 2014 (2014-11-01), pages 467-502, XP055669433, ISSN: 2168-0965, DOI: 10.7569/RAA.2014.097310**
- **SILVANI LUDOVICA ET AL: "Arabinogalactan and hyaluronic acid in ophthalmic solution: Experimental effect on xanthine oxidoreductase complex as key player in ocular inflammation (in vitro study)", EXPERIMENTAL EYE RESEARCH, ACADEMIC PRESS LTD, LONDON, vol. 196, 4 May 2020 (2020-05-04), XP086177802, ISSN: 0014-4835, DOI: 10.1016/J.EXER.2020.108058 [retrieved on 2020-05-04]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/715, A61K 2300/00;**
**A61K 31/728, A61K 2300/00**

## Description

### Field of the invention

The present invention relates to the medicalpharmaceutical field, and more precisely to preparations for topical ophthalmic use (eye drops), in particular an ophthalmic composition comprising a hyaluronic acid and arabinogalactan association. The invention also relates to an ophthalmic composition in combination with panthenol, vitamin E-D-α-polyethyleneglycol (TGPS) and trehalose. Both compositions can be applied to the ocular surface in order to ensure a significant and lasting reduction of dry-eye syndrome, and of the general inflammatory ocular state, with good local tolerability.

### Background of the invention

Dry-eye syndrome, or kerato conjunctivitis sicca, is a common eye disease which affects millions of people, caused by chronic dehydration of the conjunctiva and cornea, which causes irritation. It is mainly due to a quantitative reduction or qualitative alteration of the tear film which covers the eye and normally lubricates and protects it. Poor production or excessive evaporation of tears may be a complication of blepharitis, conjunctivitis (including allergic forms) and other inflammatory eye diseases. Dry-eye syndrome can also be the result of systemic diseases such as Sjögren's syndrome, systemic lupus erythematosus and rheumatoid arthritis. Furthermore, the disorder is typical in advanced age (due to tear gland atrophy), in women experiencing menopause (due to new hormonal balances) and in those with prolonged use ofcontact lenses, eye drops or certain systemic drugs (antihypertensives, anxiolytics, sleeping pills, antihistamines and many others).

Without going into the details of the etiology thereof, it has been shown that dry-eye syndrome and anomalies in the corneal tear film, including subsequent inflammatory changes in the entire ocular surface, are able to affect the whole eye including ocular adnexa, conjunctiva and cornea.

Although often regarded as a secondary problem, kerato conjutivitis sicca, commonly referred to as dry-eye syndrome, is becoming a growing health problem, affecting 170 of women and 11% of men in the United States alone (Burgalassi et al., 2011. Curr Eye Res. 36(1) :21-8). These data are underestimated when considering patients who do not undergo treatment and those who self-treat and/or have intermittent symptoms and mild symptoms.

Recently, the International Dry Eye Workshop (DEWS) defined dry-eye syndrome as "a multifactorial tear and ocular surface disease which results in symptoms of discomfort, visual disturbances, and tear instability with potential damage to the ocular surface which is accompanied by increased osmolarity of the tear film and inflammation of the ocular surface".

The role of an inflammatory component in dry-eye syndrome is known (Baudoin et al., 2018. Acta Ophthalmol 96(2): 111-119; Vazirani, 2018. Indian J Ophthalmol 66(1): 44; Yagci and Gurdal, 2014. Int Ophthalmol. 34 (6) :1291-1301). In fact, several biochemical factors are known, specifically some enzymes which are behind this phenomenon, such as the enzymatic cascade of cyclooxygenases (COX), to which the prostaglandin family belongs, and other enzymes such as the enzymatic complex of xanthine oxidoreductase and xanthine oxidase, present in the human cornea (Cejkova et al., 2007. Histol Histopathol 22(9):997-1003;).

In particular, xanthine dehydrogenase + xanthine oxidase is an enzymatic system which catalyzes the oxidation of hypoxanthine to xanthine, consequently producing uric acid. The enzymatic complex exists in two separate but interconvertible forms, xanthine dehydrogenase and xanthine oxidase, which generates/generate reactive oxygen species (ROS), aggravating factors of tissue damage, such as occurs in myocardial tissue following the ischemic or reperfusion phenomenon. However, this pathological situation is not limited to myocardial tissue but is present in other tissues and/or organs such as, for example, the ocular tissues and in particular the cornea (Cejkova et al., 2007. Histol Histopathol 22(9):997-1003; Kuppusamy and Zweier, 1989 The Journal of biological chemistry 264:9880-9884; Sery and Petrillo, 1984. Journal of current eye research 1:243-252; Pustivrh et al., 2000. Reproduction, fertility and development 12(6):269-275).

In particular, it has been shown that xanthine oxidase plays a key role in inflammatory processes and cytotoxicity triggered by the production of superoxide anion and also for the conversion thereof into oxygen peroxide. In fact, it has been shown that XO is an enzyme involved in dry-eye syndrome, in particular in oxidative reactions on the ocular surface of patients affected by Sjögren's syndrome with dry-eye syndrome.

### Background art

With the recognition of the role of inflammation in dry-eye syndrome, several new treatments have been studied with the aim of inhibiting the various inflammation pathways. The current medications used, which have shown an effective role in managing the syndrome, include cyclosporine A, corticosteroids, *tracolimus,* derivatives of tetracyclines

and autologous serum.

However, it should once again be emphasized that dry-eye syndrome is a multifactorial disease, on the one hand linked to the instability of the tear film and hyperosmolarity, which can be considered physicalchemical parameters, and on the other to more biological parameters such as inflammation.

Patent document WO 2009/018060 describes an ophthalmic composition comprising one or more terpene compounds, and one or more natural polymers selected from the group comprising hyaluronic acid, chondroitin sulfate, alginate, guar gum, fructan, arabinogalactan or any of the corresponding salts or derivatives thereof. In particular, the document does not take into account the simultaneous use of hyaluronic acid and arabinogalactan, and the field of application thereof remains limited to the use of the ophthalmic composition as a liquid for the care and disinfection of contact lenses or for eye drops for irritated eyes. There is no reference to either use in the treatment of dry-eye syndrome or the combined use of hyaluronic acid and arabinogalactan.

European patent EP 1912707 relates to an ophthalmic composition containing a mucus adhesive polysaccharide capable of promoting the reepithelialization of the cornea. More specifically, an ophthalmic solution containing arabinogalactan, to be used as artificial tears to stimulate the recovery of corneal lesions, especially for people who use contact lenses.

Also in this case the field of application does not take into consideration dry-eye syndrome, neither from the point of view of the chemical-physical parameters, such as the instability of the tear film and hyperosmolarity, nor of the biological parameters, such as inflammation. Furthermore, the composition does not include the presence of hyaluronic acid as an active ingredient.

Although the multifactoriality of dry-eye syndrome is known and the studies on the delicate regulatory balance of corneal tear film stability have clarified the etiology and role of the inflammatory component in the course of the disease, the need remains to date for an ophthalmic formulation for use in the treatment of dry-eye syndrome which considers both tear film instability and the role of inflammation.

Furthermore, in the literature it is known that some plants commonly used by Native Americans have healing effects: in particular, it has been shown that extracts of some plants such as *Larix laricina* affect xanthine oxidase activity (Owen and Johns, 1999. J Ethnopharmacol. 64(2):149-160). However, it should be emphasized that the use of Larix laricina extracts has been proposed to a limited extent for the treatment of gout, with an exclusively oral formulation and administration, and without any indication of the anti-inflammatory effect in the broad sense.

Furthermore the article "Mucoadhesive Polymers for Enhancing Retention in Ocular Drug Delivery: A Critical Review" (Anubha Khare et al, 2014) gives an overview of various mucoadhesive polymers used to increase the bioavailability, precorneal residence time and controlled release with reduced dosing frequency without causing any visual disturbances; also in this case the use in combination of hyaluronic acid and arabinogalactan is not disclosed.

The present invention proposes for the first time the use of hyaluronic acid in combination with arabinogalactan in an ophthalmic composition for the treatment of dry-eye syndrome, accompanied by inflammatory phenomena, linked at least in part to the action of the enzyme system xanthine dehydrogenase/xanthine oxidase, the activity of which is unexpectedly reduced thanks to the combined action of the two active ingredients: hyaluronic acid and arabinogalactan. From the same combined action of these two active ingredients comes the fact that the factors underlying the inflammatory phenomena are also reduced, at least in part.

## Summary of the invention

A first object of the present invention is the formulation of an ophthalmic composition comprising hyaluronic acid and arabinogalactan, for use in the treatment of dry-eye syndrome.

Another object of the invention is the combination of hyaluronic acid and arabinogalactan with the addition of panthenol, vitamin E-D-$\alpha$-polyethylene glycol (TGPS) and trehalose, for ophthalmic compositions in the form of a hypotonic aqueous solution, with neutral pH and without preservatives, for use in the treatment of dry-eye syndrome.

It is another object of the present invention to provide an ophthalmic composition based on hyaluronic acid and arabinogalactan, with the addition of panthenol, vitamin E-TGPS and trehalose for the treatment of dry-eye syndrome even if accompanied by inflammatory phenomena which are often connected thereto.

It is another object of the present invention to provide an ophthalmic composition based on hyaluronic acid and arabinogalactan, with the addition of panthenol, vitamin E-TGPS and trehalose, which for the addition of a gelling agent, such as Poloxamer 407, is in the form of a gel, for the treatment of dry-eye syndrome even if accompanied by inflammatory phenomena which are often connected thereto.

It is another object of the invention to provide the treatment of an inflammatory process dependent, at least in part, on the activity of the enzyme system xanthine dehydrogenase-xanthine oxidase by means of the topical application of the ophthalmic composition described herein comprising hyaluronic acid and arabinogalactan, and possibly also panthenol, vitamin E-TGPS and trehalose.

## Detailed description of the invention

The present invention relates to an ophthalmic composition based on hyaluronic acid and arabinogalactan for use in the protection, hydration, and lubrication of the eyes. Furthermore, the object of the present invention is an ophthalmic composition based on hyaluronic acid and arabinogalactan for the treatment of dry-eye syndrome, and the inflammatory state often associated therewith. According to a preferred embodiment, the composition of the present invention is a hypotonic aqueous ophthalmic solution with neutral pH and free of preservatives. In addition to hyaluronic acid and arabinogalactan, it further comprises panthenol, trehalose and Vitamin E-TGPS.

In literature there are several ophthalmic compositions for the treatment of dry-eye syndrome, but none of them includes a composition based on hyaluronic acid and arabinogalactan, comprising panthenol, vitamin E-TGPS and trehalose. Furthermore, ophthalmic compositions are not currently disclosed which provide an ophthalmic formulation for use in the treatment of dry-eye syndrome which considers both the chemical-physical parameters of the syndrome, such as tear film instability and hyperosmolarity, as well as biological ones such as the role of inflammation, and in particular the activity of the xanthine oxidase system.

Patent documents WO 2009/018060 and EP 1912707 describe, respectively, an ophthalmic composition comprising one or more terpene compounds, and one or more natural polymers (selected from the group comprising hyaluronic acid, chondroitin sulfate, alginate, guar gum, fructan, arabinogalactan or any of the corresponding salts or derivatives thereof) and an ophthalmic composition capable of promoting the reepithelialization of the cornea containing arabinogalactan.

However, dry-eye syndrome is not included in the field of application thereof, neither from the point of view of chemical-physical parameters such as tear film instability and hyperosmolarity, and of more biological parameters such as inflammation.

As reported above, the use of extracts of *Larix laricina* for the treatment of gout is known in the literature (Owen and Johns, 1999. J Ethnopharmacol. 64(2) :149-160) . This indication proposed only and exclusively for the treatment of gout does not take into consideration the ophthalmic district, nor does it consider anti-inflammatory effects.

On the other hand, the authors of the present invention have evaluated, for the first time, on the basis of the experimental tests reported below, the potential capacity of arabinogalactan and hyaluronic acid, first individually and then in association with each other with a synergistic effect, to interfere with the activity of xanthine oxidase, for the purpose of a curative/soothing ophthalmic use in syndromes with an inflammatory component which depends at least in part on the presence of xanthine oxidase.

In detail, a first object of the present invention is an ophthalmic solution based on hyaluronic acid and arabinogalactan and the synergistic effect thereof in the inhibition of xanthine oxidase.

Another object of the invention is an ophthalmic composition in which the association of hyaluronic acid and arabinogalactan is accompanied by panthenol, trehalose and Vitamin E-TGPS.

A first of the fundamental and characterizing components of the ophthalmic solution according to the present invention is hyaluronic acid, in the form of sodium hyaluronate, an anionic glucosaminoglycan widely distributed in connective, epithelial and neuronal tissues. It can be a very large molecule with a variable size depending on the tissue and body area. For example, the hyaluronic acid present in the human synovium is on average 7 million Dalton (Da) per molecule, or about 20,000 disaccharide monomers, while in other areas the size thereof is less than about 3-4 million Da. Hyaluronic acid is essentially a long chain of carbohydrates capable of recruiting and binding water molecules in a ratio equal to 1000 times the weight thereof.

Given that ocular tissues require optimal fluid levels for the proper physiology and functioning thereof, it is not surprising that hyaluronic acid forms 950 of the fluids within the eye, supporting the health of the ocular structures such as the cornea and retina. As hyaluronic acid is a polysaccharide with properties to preserve and retain water, it offers high hydration and lubrication of the corneal surface.

The chemical structure thereof is as follows:

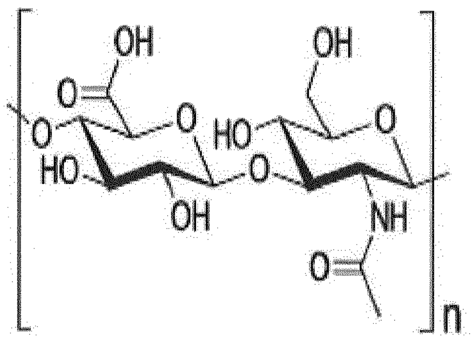

**Hyaluronic (HA) disaccharide** (composed of d-glucuronic acid and N-acetyl-d-glucosamine)

The concentration of hyaluronic acid in the solutions according to the present invention ranges from 0.1% w/w to 0.5% w/w, and more preferably from 0.15% w/w to 0.3% w/w.

A second fundamental and characterizing component of the solutions of the present invention is arabinogalactan, a natural mucopolysaccharide extracted from the bark of the Canadian Larch, even if in nature a second class of arabinogalactan derived from microbial populations can be found. It is a biopolymer consisting of the monosaccharides arabinose and galactose. Arabinogalactan exhibits non-viscous Newtonian behavior, as shown by the fact that a concentration as high as 10% w/w has a viscosity of 1.6 mPa.s, and has excellent mucus adhesive properties, useful for permanence on the ocular surface.

It is known in the literature that arabinogalactan with a concentration of at least 5% w/w exerts a good protective effect against the appearance of dry corneal areas. Furthermore, it significantly reduces the healing time of an experimental corneal lesion as early as 27 hours after the first treatment. It can therefore be considered a therapeutic product for the protection of dry-eye syndrome and for the treatment of corneal wounds (1,2,3).

However, the concentration of arabinogalactan in the solutions according to the present invention ranges from 0.30% w/w to 1.00% w/w, and more preferably from 0.4% w/w to 0.8% p/p. The chemical structure thereof is as follows:

**Arabinogalacta**

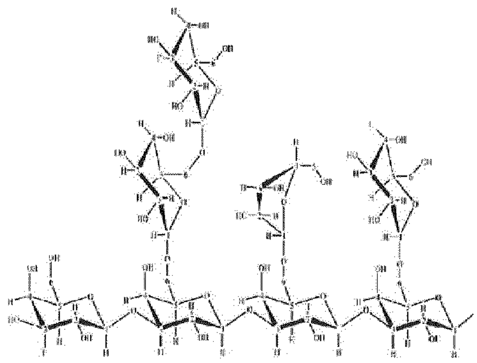

Furthermore, the ophthalmic composition of the present invention includes D-panthenol as a component. The latter is an alcohol analogue of pantothenic acid (Vitamin B5), and therefore can be considered as provitamin B5. It is rapidly oxidized to pantothenic acid in organisms. D-panthenol at room temperature is a clear viscous liquid and is used in cosmetic and drug products as a moisturizer and to improve wound healing. Sometimes conditions such as dry-eye syndrome can cause abrasions; in particular, those associated with the cornea induce particularly annoying symptoms such as tearing, redness, pain, stiffness, and blurred vision. The study conducted by Islam Mahmoud Hamdi (Effect of D-Panthenol on corneal epithelial healing after surface laser ablation. 2018. Journal of Ophthalmology) demonstrated a positive effect of panthenol on the healing of the corneal epithelium after laser ablation. The chemical structure thereof is as follows:

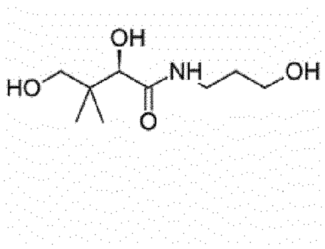

**Panthenol**

Panthenol rapidly penetrates into the skin and mucous membranes (including the intestinal membrane), where it is rapidly oxidized to pantothenic acid. This acid is particularly hygroscopic, in fact it binds water very effectively. It is also used in the biosynthesis of coenzyme A, which plays a fundamental role in a wide range of enzymatic reactions and in cell growth. Panthenol is present in the two D-/Lenantiomers thereof, but only D-panthenol (dexpanthenol) is biologically active. However, both forms have moisturizing properties. For cosmetic use, panthenol can be either in the D- form thereof, or as a racemic mixture D-/L- (DL-panthenol).

The concentration of panthenol in the solutions according to the present invention ranges from 0.150% w/w to 0.80%

w/w, and more preferably from 0.30% w/w to 0.5% w/w.

Another ingredient characterizing the ophthalmic composition of the present invention is trehalose, a disaccharide with protective and stabilizing properties of cell membranes, as well as antioxidant. Trehalose is formed from two monomers of α-glucose by means of a 1,1-glycosidic bond, giving it the name α-D-glucopyranosyl-(1→1)-α-D-glucopyranoside. This bond makes trehalose very resistant to acid hydrolysis, and therefore it is stable in solution at high temperatures, even in strongly acidic conditions.

The chemical structure thereof is as follows:

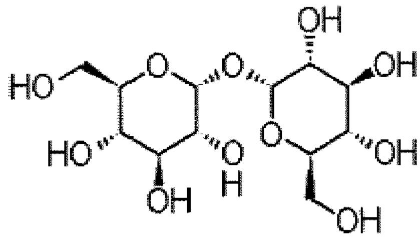

**Trehalose**

Furthermore, trehalose has remarkable water retention abilities, it safeguards the lipid bilayer during dehydration and rehydration by replacing water, forming hydrogen bonds between the -OH groups thereof and the groups of the lipid heads. Several studies (Tange t al. 2007. J Magn Resin 2007 184(2):222-227; Li et al. 2012. Molecular Vison 16:317-329) have demonstrated the efficacy and safety of trehalose as ophthalmic drops for the treatment of moderate to severe dry-eye syndrome.

The concentration of trehalose in the solutions according to the present invention ranges from 2.00% w/w to 5% w/w, and more preferably from 3% w/w to 4% w/w.

The ophthalmic composition of the present invention further includes Vitamin E-TGPS as a component, capable of stabilizing the tear film, reducing the symptoms of dry eyes, and maintaining natural hydration. Furthermore, it assists the healing process and shows a strong antioxidant power, once it has been hydrolyzed by the effect of esterases present in the tear fluid, especially in inflammatory syndromes (17). In addition, it improves the protection of the epithelium cells of the cornea and conjunctiva, decreasing the risk of inflammatory phenomena at the same time. The chemical structure thereof is as follows:

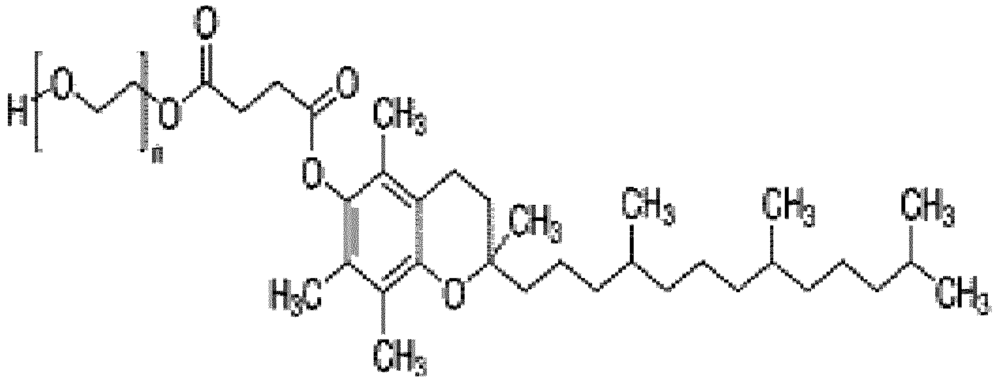

ranges from 0.01% w/w to 0.1% w/w, and more preferably equal to 0.030% w/w.

In yet another aspect of the present invention, the treatment of dry-eye syndrome is described by means of the topical application of the ophthalmic composition described herein. Thus the invention provides a composition for use in a method for the treatment of the dry-eye syndrome condition which comprises the administration of an effective therapeutic amount of a composition comprising hyaluronic acid and arabinogalactan comprising panthenol, trehalose and vitamin E-TGPS in such amounts and for a time such as to induce the desired effect.

The compositions are administered in any amount and by any route of administration suitable for ocular treatment. The exact dosage will be chosen by the physician according to the state of the patient to be treated. Dosage and administration will be tailored to provide sufficient active ingredient levels or to maintain the desired effect. Additional factors which may be considered comprise the severity of the condition, course of the disease, age, weight and gender

of the patient, administration timing and frequency, diet, combination with other drugs, sensitivity reactions, tolerance and response to therapy.

The active ingredients and components of the invention are formulated in a form suitable for topical application in the eye, preferably in the form of eye drops, instillable in drops, each drop consisting of a form of dosage unit so as to allow easy administration and dosage uniformity.

It is understood how the dry-eye syndrome treatment method according to the present invention is not limited in the use thereof in humans, but the use thereof can also be extended to all mammals.

**Pharmaceutical forms and dosages of the composition in object**

Pharmaceutical compositions

Pharmaceutical compositions comprising hyaluronic acid and arabinogalactan are provided in one aspect of the present invention. Compositions further comprising panthenol, vitamin E-TGPS and trehalose are also provided. In some embodiments such compositions may comprise one or more additional therapeutic agents, selected from the group comprising growth factors, anti-inflammatory agents, vitamins, fibronectin and collagen, amino acids, polysaccharides and various buffer systems such as phosphate, citrate and tris.

The ophthalmic composition according to the present invention can be prepared in any pharmaceutical form which allows the corneal application thereof, such as eye drops, gels, ointments, but preferably it is prepared in the form of eye drops sterile by filtration at 0.2 microns. This is because the sterilization with flowing steam at 121°C for at least 15 minutes, as prescribed by the pharmacopoeia, is deleterious for the stability of the eye drops, especially with regard to some components such as vitamins and hyaluronic acid.

The ophthalmic composition may comprise one or more additional therapeutic agents, selected from the group comprising growth factors, anti-inflammatory agents, vitamins, fibronectin and collagen, amino acids, polysaccharides, and various buffer systems such as phosphate, citrate, and tris.

**EXAMPLES**

Basic formulations are now reported for illustrative purposes, without however being a limitation to the invention. Other aspects, advantages, or modifications within the scope of the invention will become apparent to those skilled in the art pertaining to the invention.

**BASIC formula according to the invention: hyaluronic acid and arabinogalactan association and borate buffer.**

The basic formula of the eye drops according to the present invention is reported in the following Table 1:

**Table 1.** Basic eye drops formula

| BASIC formulation | % w/w |
|---|---|
| Hyaluronic acid sodium salt | **0.15** |
| Arabinogalactan | **0.40** |
| Boric Acid | **0.852** |
| Sodium tetraborate decahydrate | **0.137** |

According to an aspect of the invention, the BASIC formula of Table 1 has been suitably implemented with some specific ingredients to obtain a formulation in the form of eye drops as reported in Table 2, which forms a preferred embodiment of the invention.

**Table 2.** Preferred formula of the five-component eye drops (hyaluronic acid and arabinogalactan association + panthenol, trehalose, and Vitamin E-TGPS)

| Basic formulation Eye drops | % w/w | Action | # CAS |
|---|---|---|---|
| Hyaluronic acid sodium salt | 0.15 - 0.4 | Viscous moisturizing agent | |
| Arabinogalactan | 0.40 - 1.00 | Immunostimulating reepithelialization agent | |

(continued)

| Basic formulation Eye drops | % w/w | Action | # CAS |
|---|---|---|---|
| Panthenol | 0.30 - 0.60 | Anti-inflammatory emollient | |
| Trehalose | 3.00 - 4.00 | Membrane stabilizing agent and osmolality | |
| Vitamin E-TGPS | 0.030 | Antioxidant stabilizing the tear film | |
| Boric Acid | 0.852 | Buffer | |
| Sodium tetraborate decahydrate | 0.137 | Buffer | |
| Distilled water | Up to 100 | | |
| Sterile by filtration **0.2μ** | Yes | | |

Following the experimentation carried out, the formulation of particularly preferred eye drops according to the invention was defined, as reported in the following Table 3. These eye drops are identified below by the letter **A.**

**Table 3.**

| Effective formulation of the eye drops **A** | |
|---|---|
| Formulation A | % w/w |
| Hyaluronic acid sodium salt | 0.15 |
| Arabinogalactan | 0.40 |
| Panthenol | 0.30 |
| Vitamin E-TGPS | 0.030 |
| Trehalose | 3.0 |
| Boric Acid | 0.852 |
| Sodium tetraborate decahydrate | 0.137 |
| Osmolality mOsm/kg | **240-270** hypotonic |
| Sterile by filtration at **0.2 μ** | Yes |

The solution thus obtained is clear in appearance, has a neutral pH ranging from 6.8 to 7.6, and an osmolality in the range of 240-270 mOsm/kg, thus resulting slightly hypotonic.
Said solution is then easily sterilized by filtration (0.2 microns), also in consideration of the action of the arabinogalactan which seems to dilute the hyaluronic acid, making the eye drops less viscous, albeit in a limited way.

**Preparation method**

The preparation method is not particular in itself, and involves steps known to those skilled in the art. However, as is known to those skilled in the art, normally when polysaccharide structures are mixed with hyaluronic acid, a common increase in viscosity is found.
Surprisingly, the solubilization of the various components of the eye drops of the invention is not difficult. The presence of arabinogalactan makes the solution particularly easy to filter at 0.2 um, as the latter has the ability to decrease the viscosity of the hyaluronic acid and therefore also of the final eye drops, avoiding possible expected increases of the viscosity.
Finally, according to another aspect of the invention, with the addition of a gelling agent such as Poloxamer 407 for a concentration range between 1% and 9% by weight, to the solution having the composition of Table 3, a gel was obtained before sterilization by filtration, which was much more viscous than the eye drops, still for ophthalmic use, while maintaining the synergistic effects of the AG + HA association, on the xanthine oxidase activity.

## EXPERIMENTAL PART

As noted above, dry-eye syndrome is a multifactorial syndrome with a marked inflammatory component. Some enzymes, such as COX and XO, which reside upstream of the inflammatory cascade are strongly involved in the course of the syndrome; in particular, XO is an enzyme involved in dry-eye syndrome in patients with Sjorgen's syndrome.

Starting from the above considerations, the experimental activity first involved the evaluation of the potential capacity to interfere with the xanthine oxidase activity of the BASIC formulation containing hyaluronic acid and arabinogalactan in borate buffer.

In particular, arabinogalactan, probable constituent of larch extracts, proposed as anti-gout agents, and therefore possible XOD activity inhibitors, was evaluated. In fact, this evaluation was carried out for arabinogalactan, for hyaluronic acid and for the association thereof.

The enzymatic reaction, which is reported for clarity, consists of:

**Xanthine oxidase + Xanthine → Uric acid**

Arabinogalactan or hyaluronic acid or arabinogalactan with hyaluronic acid were added to this reaction. Given the positive results, this evaluation was extended to the eye drops formulation in the entirety thereof comprising panthenol, vitamin E-TGPS and trehalose. Also in this case the same xanthine oxidase activity inhibition was obtained, confirming the anti-inflammatory potential of the eye drops of the present invention.

Furthermore, since the product of xanthine oxidase is both uric acid and superoxide anion $O_2^{-\cdot}$, the ability to inhibit the formation of the latter was evaluated for arabinogalactan, for hyaluronic acid and for the association thereof.

Any inhibition is particularly important because the superoxide anion production is at the origin, at least in part, of the inflammatory phenomena of the ocular surface. The superoxide anion produced by xanthine oxidase in the oxidation reaction of xanthine was detected by the reduction of **ferrocytochrome c** to **ferrocytochrome c** by the same superoxide anion ($O_2^{-\cdot}$) (Fridovich). The reaction is reported here for clarity:

**$Fe^{3+}$Cit.C + Superoxide Anion -----→$Fe^{2+}$Cit.C**

Arabinogalactan or hyaluronic acid or arabinogalactan with hyaluronic acid were added to this reaction. Furthermore, given the positive results, this evaluation was extended to the eye drops formulation in the entirety thereof comprising panthenol, vitamin E-TGPS, trehalose. Also in this case the same xanthine oxidase activity inhibition was obtained, confirming the anti-inflammatory potential of the eye drops of the present invention.

Finally, chemical-physical parameters characterizing the formulation of the ophthalmic composition of the present invention were evaluated, by carrying out rheology measurements. This was done to explain and confirm the interaction between arabinogalactan and hyaluronic acid as much as possible. Specifically, the chemical-physical rheological parameters analyzed were the storage modulus g' (elastic modulus) with the loss modulus g" (viscous modulus) and viscosity (pa.s) as such.

All with the aim of characterizing the arabinogalactan + hyaluronic acid association and the eye drops derived therefrom and of the present invention, containing, in addition to arabinogalactan and hyaluronic acid, panthenol, vitamin E-TGPS, and trehalose.

**Verification of the xanthine oxidase (XOD) reaction inhibition and formation of uric acid Materials and Methods**

The effect of arabinogalactan, hyaluronic acid and the association thereof was investigated by monitoring the formation of uric acid over time (0, 1, 5, 10, 15, 30 min). Uric acid concentration was determined at 291 nm using a DR6000 UV spectrophotometer (Hach, Lange). The absorbance value is related to the concentration by means of the Lambert-Beer law:

$$A = \varepsilon b C$$

where A is the absorbance of uric acid at 291 nm, ε is the molar absorption coefficient, b is the optical path (thickness of the cuvette in which the solution is contained) and C is the uric acid concentration.

The blank/control consists of: 50 μM xanthine, 10 mg $L^{-1}$ of xanthine oxidase (XO) (0.00016 μUI of enzyme) in borate buffer solution (see table 3) $7.2 \leq pH \leq 7.8$). The effect of the components of the eye drops was investigated by adding the individual components to the blank solution: respectively

**(i)** 0.40 (w/w) of arabinogalactan (AG)

**(ii)** 0.15% (w/w) of hyaluronic acid (HA)
**(iii)** association of 0.4%(w/w) of AG +
0.15% (w/w) of HA (mix AG + HA).
**(iiii)** Eye drops corresponding to the formulation of Tab.3.

All the tests were conducted at least in triplicate.
The uric acid formation reductions were calculated as:

$$Reduction\,\% = 100 - (\frac{C_{30-i}}{C_{30-control}} * 100)$$

where $C_{30-(i)}$ is the final concentration (30 min) of uric acid in the i-th test and $C_{30\text{-control}}$ is the uric acid concentration at 30 min in the blank (control).
Furthermore, the inhibition evaluation reported above was also carried out in borate buffer for arabinogalactan, for hyaluronic acid and for the association thereof, and finally for the eye drops formulation of the present invention, also comprising panthenol, vitamin E- TGPS and trehalose.

## Results and Discussion

The results are summarized in Table 4 and in the graphs below.

**TABLE 4**

Inhibition of xanthine oxidase activity: Inhibition of uric acid production

| | Blank | i | ii | iii |
|---|---|---|---|---|
| | XOD reaction Uric acid µM | XOD + Arabinogalactan reaction Uric acid µM | XOD + Hyaluronic Acid reaction Uric acid (µM) | XOD + Arabinogalactan reaction + Hyaluronic acid Uric acid |
| R2* | 0.93 | 0.99 | 0.99 | 0.91 |
| Inhibition % | - | 27 | 0 | 39 |
| µM produced in 30 minutes | 15.3 | 11.4 | 15.3 | 10 |
| µM produced per minute | 0.51 | 0.38 | 0.51 | 0.35 |

- R2 was calculated assuming/approximating a linear oxidation kinetics of xanthine to uric acid.

  T minutes

Average of three determinations

The AG and AG/HA association cause a statistically significant decrease in uric acid compared to the blank, respectively 26%, 39%. In particular, as can be seen in Fig. 1b, the presence of hyaluronic acid has no effect on the formation of uric acid (0% inhibition). On the other hand, the fact that the percentage of uric acid is reduced by the AG+HA association to a greater extent than the solution containing the AG alone suggests a possible synergistic effect on the inhibition by AG and HA against the enzyme xanthine oxidase or a possible interaction block between the enzyme xanthine oxidase and the substrate thereof, xanthine.
Due to the shape thereof, arabinogalactan could have a sequestering capacity for the enzyme xanthine oxidase and/or could mask the substrate (xanthine) to the enzyme. It has been observed that the combination of arabinogalactan and hyaluronic acid exerts and enhances the same effect regardless of the inhibition mechanism. This has its own

experimental validity and an unexpected and innovative statistical significance.

Moreover, the same results were also obtained when the eye drops formulation of the present invention was evaluated, comprising, in addition to arabinogalactan and hyaluronic acid, also panthenol, vitamin E-TGPS and trehalose. The results confirmed the same inhibitory capacity: in fact, it goes from an inhibition of 38% in the case of the hyaluronic acid + arabinogalactan association to a slightly higher value, specifically 45%.

Table 5 follows.

**Table 5**

Inhibition of xanthine oxidase activity: inhibition of uric acid production due to the effect of the eye drops. (**iii**)

| | XOD reaction Uric acid μM | XOD reaction + Eye drops Uric acid μM |
|---|---|---|
| R2* | 0.93 | 0.99 |
| Inhibition % | - | 45 |
| μM produced in 30 minutes | 15.3 | 8.4 |
| μM produced per minute | 0.51 | 0.28 |

Verification of superoxide anion production inhibition due to the effect of xanthine oxidase

## Materials and Methods

The effect of arabinogalactan, hyaluronic acid, and the association thereof on superoxide anion production over time (0, 1, 5, 10, 15, 30 min) was investigated. The production of superoxide anion was determined by the reduction of **ferrocytochrome C (3+)** to **ferrocytochrome C (2+),** monitoring absorption at 551 nm using a DR6000 UV spectrophotometer (Hach, Lange). The tests for the determination of superoxide anion production were carried out still using the xanthine oxidase reaction used previously.

Produced by the superoxide anion, the ferrocytochrome C was calculated considering a molar absorption equal to $2.1 \times 10^4$ $M^{-1}.cm^{-1}$ (Ref.16-17). The superoxide inhibition evaluation reported above was performed for arabinogalactan, hyaluronic acid, and the association thereof. Furthermore, the eye drops formulation of the present invention was also evaluated, also comprising panthenol, vitamin E-TGPS and trehalose.

## Results and Discussion

The data on the inhibition capacity of superoxide anion production are summarized in Table 6 and in the graphs relating to the inhibition kinetics, shown in the accompanying figures in which:

figures 1a-c relate to the kinetics of uric acid formation, with the uric acid concentration [μM] vs time [min] in the absence of components (control/blank) and in the presence of (a) arabinogalactan (AG), (b) hyaluronic acid (HA) and (c) arabinogalactan and hyaluronic acid (AG + HA);

Figures 2a-c relate to the kinetics of ferrocytochrome C formation, with the absorption of the ferrocytochrome [abs] vs time [min] in the absence of components (control/blank) and in the presence of: (a) arabinogalactan (AG), (b) hyaluronic acid (HA) and (c) arabinogalactan and hyaluronic acid (AG + HA);

fig. 3 is a graph of the trend of G" **(Loss Modulus** Viscosity) of hyaluronic acid 0.15%, hyaluronic acid 0.15% + arabinogalactan 0.4% and arabinogalactan at 0.4%.

**TABLE 6**

| | Blank | i | ii | iii |
|---|---|---|---|---|
| | Control Production $Fe^{+2}$ Cit.C | Reaction with Arabinogalactan Production $Fe^{+2}$ Cit.C | Reaction with Hyaluronic acid Production $Fe^{+2}$ Cit.C | Reaction with Arabinogalactan + Hyaluronic Acid Production $Fe^{+2}$ Cit.C |
| $R^{2*}$ | 0.99 | 0.99 | 0.74 | 0.98 |
| Inhibition % | 0 | 38.1 | 17.2 | 61.8 |
| µM produced in 30 minutes | 15.3 | 9.3 | 12.6 | 5.91 |
| µM produced per minute | 0.51 | 0.31 | 0.42 | 0.197 |

From the data obtained it is clear that the inhibition of xanthine oxidase activity, by arabinogalactan, hyaluronic acid and even more by the association thereof, has a first confirmation in the inhibition of uric acid, but also has a second, even more significant and important finding in the inhibition of superoxide anion production. Furthermore, referring to the inhibition of superoxide anion production, it is noted how the addition of hyaluronic acid to arabinogalactan, which is undoubtedly the major architect of the inhibitory effect, increases this inhibitory effect. What is reported, however it can be explained, whatever the underlying mechanism is, is new and useful from the pharmacological point of view, with reference to inflammatory phenomena in which there is even partial participation of xanthine oxidase.

Furthermore, the same results were also obtained when the eye drops formulation of the present invention was evaluated, comprising, in addition to arabinogalactan and hyaluronic acid, also panthenol, vitamin E-TGPS and trehalose. The results reported in Table 7 confirmed the same inhibitory capacity.

**TABLE 7**

| | Control $Fe^{+2}$ Cit.C production | Reaction with Eye drops $Fe^{+2}$ Cit.C production |
|---|---|---|
| $R^{2*}$ | 0.99 | 0.98 |
| Inhibition % | 0 | 65.0 |
| µM produced in 30 minutes | 2.25 | 1.02 |
| \ | 0.098 | 0.034 |

**Rheological characterization of the formulation of the present invention.**

In addition to the evaluation of the effect of arabinogalactan, hyaluronic acid and the association thereof on xanthine oxidase activity and in order to characterize the possible interaction thereof, to justify the inhibitory effect thereof, rheology measurements were carried out with the hypothesis that the two macrostructures, arabinogalactan on the one hand and hyaluronic acid on the other, could modify the rheological trend of the formulation of the present invention.

**MATERIALS AND METHODS**

With reference to fig. 3, the characterization of the following solutions was carried out using an AM Instruments Plate Viscometer, in Oscillation Mode. The following solutions were measured:

**Sample 1:** Hyaluronic acid solution at

0,15% w/w
**Sample 2:** Arabinogalactan solution 0.40 +
Hyaluronic acid 0.15% w/v
**Sample 3:** Arabinogalactan solution 0.4% w/w.

**First Point**

The graph shown in figure 3 shows the trend of G" (Loss Modulus viscosity) of the three solutions:

| | |
|---|---|
| Solution no. 1: | Hyaluronic acid 0.15% w/w |
| Solution no. 2: | Arabinogalactan 0.40 + hyaluronic acid 0.15% w/w |
| Solution no. 3: | Arabinogalactan 0.4% w/w |

Interpretation of the Results:

The measurements in oscillation mode related to G" indicated that:

Solution no. 1: Hyaluronic acid 0.15% w/w has a linear modulus G" (Loss Modulus Viscosity), typical of a Newtonian product, always with a value higher than the other measured solutions;
Solution no. 2: Arabinogalactan 0.4% + Hyaluronic acid 0.15% w/w has a modulus G" (Loss Modulus Viscosity) with a linear trend, typical of a Newtonian product, parallel to but slightly lower than Solution no. 1
Solution no. 3: Arabinogalactan 0.4% has a non-linear modulus G" (Loss Modulus Viscosity), always much lower than the other Solutions 1 and 2.

From what has been said, it is clear how unexpectedly the presence of arabinogalactan causes the modulus G" (viscosity) of hyaluronic acid to decrease.
The measured value can be expressed as follows:

**Hyaluronic Acid G"> Arabinogalactan + Hyaluronic Acid G" > Arabinogalactan**

**Secondo Point**

The graph in fig. 3 shows the trend of **G’** (Storage Modulus elasticity) of the three solutions:

| | |
|---|---|
| Solution no. 1: | Hyaluronic acid 0.15% w/w |
| Solution no. 2: | Arabinogalactan 0.4% + Hyaluronic acid 0.15% w/w; |
| Solution no. 3: | Arabinogalactan 0.4% w/w. |

**Interpretation of the Results:**

The measurements in oscillation mode related to G’ indicate that the three solutions have a low G’ value and therefore low elasticity value.
From the data obtained, it can be noted that the viscosity of arabinogalactan is particularly low; the molecule does not have any rheological properties such as viscosity and elasticity. Hyaluronic acid has good viscosity even if not at high concentrations and behaves like a Newtonian fluid.
The association of arabinogalactan + hyaluronic acid does not lead, as one might expect, to an increase in the viscosity of the AG + HA mixture compared to hyaluronic acid alone, but on the contrary, a slight decrease in viscosity is observed, which is surprising, since usually the addition of polysaccharides to a hyaluronic acid solution does not decrease the viscosity of the polymer but increases it or, at least, keeps it unchanged.
On the other hand, it seems that the addition of arabinogalactan has a slight diluting capacity on the hyaluronic acid concentration. This result, frankly unexpected, even if numerically not very relevant, can further support the hypothesis of a particular interaction between arabinogalactan and hyaluronic acid, confirming, albeit indirectly, the synergy between the two active ingredients already observed at the enzymatic reaction level, in particular with inhibitory capacity on the xanthine oxidase reaction.
Given the results obtained, the characterization was further extended to determine the viscosity as a function of the shear rate, always using a Plate Viscometer, in particular the following solutions were measured.

Sample 1: Hyaluronic acid
Sample 2: Hyaluronic acid + Arabinogalactan
Sample 3: Eye drops A
Sample 4: Arabinogalactan

Interpretation of the Graph (Fig. 4)

1: Hyaluronic acid: linear trend (Newtonian) higher viscosity; linear trend (Newtonian) viscosity;
2: Hyaluronic Acid + Arabinogalactan: lower than the previous one;
3: Complete eye drops: linear trend (Newtonian) viscosity lower than the two previous solutions;
4: Arabinogalactan non-linear trend (viscosity lower than the three previous solutions).

The description of the graph can be further exemplified by the following table which reports the viscosity values (mPa.s) at 10[1/s] at a fixed point corresponding to A.
Table 8 follows.

**Table 8**

| | |
|---|---|
| Sample 1 Hyaluronic Acid | 1.50 |
| Eye drops 2 Hyaluronic Acid + Arabinogalactan | 1.1 |
| Sample 3 Complete eye drops A | 0.97 |
| Sample 4 Arabinogalactan | 0.16 |

From the data obtained in table 8 it can be noted that:

- Hyaluronic acid alone has a higher viscosity (1.50 mPa.s) than the viscosity of hyaluronic acid + arabinogalactan (1.1 mPa.s): in fact, there is a decrease of 0.4 mPa.s.;

  - higher than Eye Drops A (0.97 mPa.s): in fact, there is a further decrease of 0.03 mPa.s. This very slight decrease could be ascribed to the presence of Vitamin E TPGS in the eye drops;
  - Finally, even higher than the arabinogalactan 0.16 mPa.s, which is practically non-viscous.

CONCLUSIONS

In conclusion, with the tests carried out, the inventors demonstrated for the first time that the association between arabinogalactan and hyaluronic acid has a synergistic effect in the reduction/inhibition of uric acid by the enzyme xanthine oxidase. The synergistic effect of the AG + HA association, demonstrated by the experimental part, is statistically significant and the inhibiting effect thereof is greater than that expected from the mere algebraic sum of the effects obtained for the same compounds alone.
Similarly, the same synergistic phenomenon of the AG + HA association has been observed and demonstrated in the ability to reduce superoxide anion formation. Also in this case the synergistic effect of the AG + HA association is statistically significant and is greater than that obtained by the algebraic sum of arabinogalactan and hyaluronic acid alone.
These innovative results not deducible from the state of the art are the backbone of the object of the present invention, i.e., a formulation of an ophthalmic composition comprising hyaluronic acid and arabinogalactan, with the addition of panthenol, vitamin E-TGPS and trehalose, for use in the treatment of dry-eye syndrome. The synergistic effects of the AG + HA association, synergy even maintained in the testing of the ophthalmic composition in the entirety thereof, integrates the treatment of dry-eye syndrome, also and above all (if) accompanied by inflammatory phenomena, which are often connected thereto. In fact, the action of the AG + HA association, both of inhibiting uric acid formation by xanthine oxidase, and the inhibition of superoxide anion formation, are strong indications of an anti-inflammatory power.
In particular, xanthine oxidase plays a key role in inflammatory processes, especially those associated with dry-eye syndrome; moreover, the inhibiting capacity of the superoxide anion, by the AG + HA association and by the formulation of the ophthalmic composition of the invention, is of particular importance to eliminate all those phenomena

of cytotoxicity, associated with the superoxide anion, and origin of the inflammatory state of the ocular surface.

Finally, the uniqueness and innovation of the AG + HA association, and consequently of the formulation of the ophthalmic composition based on this association and of the present invention, have been independently confirmed in parallel also by the rheological measurements, which have shown a real interactive effect between arabinogalactan and hyaluronic acid, also maintained by the preferred ophthalmic formulation of the present invention both in the form of eye drops and gel.

**REFERENCES**

1. Histol Histopathol (2002) 17: 755-760 Xanthine oxidoreductase and xanthine oxidase in human cornea

2. J. Čejková1, T. Ardan1, M. Filipec2 and A. Midelfart3 Curr Eye Res. 2011 Jan;36(1) :21-8. doi: 10.3109/02713683.2010.523193. Arabinogalactan as active compound in the management of corneal wounds: in vitro toxicity and in vivo investigations on rabbits.

3. Burgalassi S, Nicosia N, Monti D, Falcone G, Boldrini E, Fabiani O, Lenzi C, Pirone A, Chetoni P. Larch arabinogalactan for dry eye protection and treatment of corneal lesions: investigations in rabbits. J Ocul Pharmacol Ther. 2007 Dec; 23(6) :541-50.

4. Rolando M1, Valente C. Establishing the tolerability and performance of tamarind seed polysaccharide (TSP) in treating dry eye syndrome: results of a clinical study. Ophthalmol. 2007 Mar 29;7:5.

5. Islam Mahmoud Hamdi. Effect of D-Panthenol on Corneal Epithelial Healing after Surface Laser Ablation. Journal of Ophthalmology. Volume 2018, Article ID 6537413, 6 page

6. Ming Tang,a Alan J. Waring,b and Mei Honga. Trehalose-Protected Lipid Membranes for Determining Membrane Protein Structure and Insertion. J Magn Reson. 2007 Feb; 184(2): 222-227.

7. Jinyang Li,Christophe Roubeix,Yu Wang,Shuai Shi,Guoting Liu,Christophe Baudouin,Wei Chen Therapeutic efficacy of trehalose eye drops for treatment of murine dry eye induced by an intelligently controlled environmental system. Molecular Vison 2012 16 317-329

8. Christophe Baudouin, Murat Irkeç, Elisabeth M. Messmer, José M. Benitez del Castillo, Stefano Bonini Clinical impact of inflammation in dry eye disease: proceedings of the ODISSEY group meeting. Acta Ophthalmol. 2018 Mar; 96(2): 111-119.

9. Jayesh Vazirani Ocular surface inflammation in dry eye disease: What we know and what we do not. Indian J Ophthalmol. 2018 Jan; 66(1): 44.

10. Yagci A1, Gurdal C. The role and treatment of inflammation in dry eye disease.Int Ophthalmol. 2014 Dec;34(6):1291-301. doi: 10.1007/s10792-014-9969-x. Epub 2014 Jul 26.

11. Cejková J1, Ardan T, Jirsová K, Jechová G, Malec J, Simonová Z, Cejka C, Filipec M, Dotrelová D, Brunová B. The role of conjunctival epithelial cell xanthine oxidoreductase/xanthine oxidase in oxidative reactions on the ocular surface of dry eye patients with Sjögren's syndrome.Histol Histopathol. 2007 Sep;22(9):997-1003. doi: 10.14670/HH-22.997

12. P Kuppusamy and J L Zweier.Characterization of free radical generation by xanthine oxidase. Evidence for hydroxyl radical generation. The Journal of Biological Chemistry 264, 9880-9884. June 15, 1989

13. Theodore W. & Raymond Petrillo and Thomas Jefferson nSuperoxide anion radical as an indirect mediator in ocular inflammatory disease. Journal Current Eye Research Volume 3, 1984 - Issue 1 243-252

14. Carolina Pustovrh, Alicia Jawerbaum, Debora Sinner, Mario Pesaresi, Mario Baier, Paula Micone, Martha Gimeno and Elida T. Gonzalez. Membrane-type matrix metalloproteinase-9 activity in placental tissue from patients with pre-existing and gestational diabetes mellitus.Reproduction, Fertility and Development 12(6) 269 - 275 2000

15. Owen PL1, Johns T Xanthine oxidase inhibitory activity of northeastern North American plant remedies used for gout. J Ethnopharmacol. 1999 Feb;64(2):149-60.

16. Massey,V: Handbook of Methods for oxygen radical Research Third Edition 1987 p. 213-2015 Irwin Fridovich. Biock.Biophys.Acta 34,255.1959 17) Biochem Pharmacol. 1990 May 15;39(10):1597-601. Comparative evaluation of the antioxidant activity of alpha-tocopherol, alpha-tocopherol polyethylene glycol 1000 succinate and alpha-tocopherol succinate in isolated hepatocytes and liver microsomal suspensions. Carini R1, Poli G, Dianzani MU, Maddix SP, Slater TF, Cheeseman KH.

## Claims

1. An ophthalmic composition comprising hyaluronic acid and arabinogalactan in synergistic amounts for use in the treatment of dry-eye syndrome.

2. An ophthalmic composition comprising hyaluronic acid and arabinogalactan in synergistic amounts according to claim 1, for use in the treatment of dry-eye syndrome, accompanied by the inflammatory phenomena related to the action of the enzyme system xanthine dehydrogenase/xanthine oxidase, which are reduced due to the combined and synergistic action of the two active ingredients hyaluronic acid and arabinogalactan.

3. An ophthalmic composition according to claim 1 or 2, further comprising panthenol, trehalose and vitamin E-D-α-polyethylene glycol (TGPS), for use in the treatment of dry-eye syndrome, accompanied by the inflammatory phenomena related to the action of the enzyme system xanthine dehydrogenase/xanthine oxidase.

4. An ophthalmic composition for use according to any one of claims 1 to 3, **characterized in that** the hyaluronic acid amount ranges from 0.15% to 0.4% by weight, on the total weight.

5. An ophthalmic composition for use according to any one of claims 1 to 3, **characterized in that** the arabinogalactan amount ranges from 0.40% to 1.00% by weight, on the total weight.

6. An ophthalmic composition for use according to claim 3, **characterized in that** the panthenol amount ranges from 0.30% to 0.60% by weight, on the total weight.

7. An ophthalmic composition for use according to claim 3, **characterized in that** the trehalose amount ranges from 3.00% to 4.00% by weight, on the total weight.

8. An ophthalmic composition for use according to claim 3, **characterized in that** the vitamin E-D-α-polyethylene glycol (TGPS) amount is equal to 0.030% by weight, on the total weight.

9. An ophthalmic composition for use according to claim 3, **characterized in that** it is filterable and sterilizable at 0.2 microns.

10. An ophthalmic composition for use according to claim 1 or 3, further comprising one or more additional therapeutic agents, selected from the group comprising growth factors, anti-inflammatory agents, vitamins, fibronectin and collagen, amino acids and/or pharmacologically acceptable substances for the treatment of dry-eye syndrome, accompanied by the inflammatory phenomena related to the action of the enzyme system xanthine dehydrogenase/xanthine oxidase.

11. Use of a hyaluronic acid and arabinogalactan association, in synergistic amounts, for preparing, after sterilization by filtration, an ophthalmic product for topical use in the form of eye drops for the treatment of dry-eye syndrome.

12. Use of a composition comprising a hyaluronic acid and arabinogalactan association, further comprising panthenol, trehalose, and vitamin E-D-α-polyethylene glycol (TGPS), for producing eye drops for the treatment of dry-eye syndrome, after sterilization by filtration.

## Patentansprüche

1. Eine ophthalmische Zusammensetzung, umfassend Hyaluronsäure und Arabinogalactan in synergistischen Mengen zur Verwendung bei der Behandlung des Trockenen-Augen Syndroms.

2. Eine ophthalmische Zusammensetzung, umfassend Hyaluronsäure und Arabinogalactan in synergistischen Mengen gemäß Anspruch 1, zur Verwendung bei der Behandlung des Trockenen-Augen Syndroms, begleitet von den Entzündungserscheinungen, die mit der Wirkung des Enzymsystems Xanthin-10-Dehydrogenase/Xanthinoxidase zusammenhängen und die durch die kombinierte und synergistische Wirkung der beiden Wirkstoffe Hyaluronsäure und Arabinogalactan reduziert werden.

3. Eine ophthalmische Zusammensetzung nach Anspruch 1 oder 2, ferner umfassend Panthenol, Trehalose und Vitamin E-D-a-Polyethylenglykol (TGPS), zur Verwendung bei der Behandlung des Trockenen-Augen Syndroms in Begleitung von Entzündungserscheinungen, die mit der Wirkung des Enzymsystems Xanthin-Dehydrogenase/Xanthinoxidase zusammenhängen.

4. Eine ophthalmische Zusammensetzung zur Verwendung nach einem der Ansprüche 1 bis 3, **dadurch gekennzeichnet, dass** die Hyaluronsäuremenge im Bereich von 0,15 bis 0,4 Gew.-%, bezogen auf das Gesamtgewicht, liegt.

5. Eine ophthalmische Zusammensetzung zur Verwendung nach einem der Ansprüche 1 bis 3, **dadurch gekennzeichnet, dass** die Arabinogalactanmenge im Bereich von 0,40 bis 1,00 Gew.-%, bezogen auf das Gesamtgewicht, liegt.

6. Eine ophthalmische Zusammensetzung zur Verwendung nach Anspruch 3, **dadurch gekennzeichnet, dass** die Panthenolmenge im Bereich von 0,30 bis 0,60 Gew.-%, bezogen auf das Gesamtgewicht, liegt.

7. Eine ophthalmische Zusammensetzung zur Verwendung nach Anspruch 3, **dadurch gekennzeichnet, dass** die Trehalosemenge im Bereich von 3,00 bis 4,00 Gew.-%, bezogen auf das Gesamtgewicht, liegt.

8. Eine ophthalmische Zusammensetzung zur Verwendung nach Anspruch 3, **dadurch gekennzeichnet, dass** die Menge an Vitamin E-D-a-Polyethylenglykol (TGPS) gleich 0,030 Gew.-%, bezogen auf das Gesamtgewicht, ist.

9. Eine ophthalmische Zusammensetzung zur Verwendung nach Anspruch 3, **dadurch gekennzeichnet, dass** sie bei 0,2 $\mu$m filtrierbar und sterilisierbar ist.

10. Eine ophthalmische Zusammensetzung zur Verwendung nach Anspruch 1 oder 3, ferner umfassend einen oder mehrere zusätzliche therapeutische Mittel, ausgewählt aus der Gruppe umfassend Wachstumsfaktoren, entzündungshemmende Mittel, Vitamine, Fibronektin und Kollagen, Aminosäuren und/oder pharmakologisch akzeptable Substanzen zur Behandlung des Trockenen-Augen Syndroms in Begleitung von Entzündungserscheinungen, die mit der Wirkung des Enzymsystems Xanthin-Dehydrogenase/Xanthinoxidase zusammenhängen.

11. Verwendung einer Assoziation von Hyaluronsäure und Arabinogalactan in synergistischen Mengen zur Herstellung, nach Sterilisierung durch Filtration, eines ophthalmischen Produkts zur topischen Anwendung in Form von Augentropfen zur Behandlung des Trockenen-Augen Syndroms.

12. Verwendung einer Zusammensetzung, die eine Hyaluronsäure- und Arabinogalactan-Assoziation umfasst, ferner umfassend Panthenol, Trehalose und Vitamin E-D-a-Polyethylenglykol (TGPS), zur Herstellung von Augentropfen für die Behandlung des Sicca-Sydroms, nach Sterilisation durch Filtration.

## Revendications

1. Composition ophtalmique comprenant de l'acide hyaluronique et de l'arabinogalactane en quantités synergiques pour son utilisation dans le traitement du syndrome de l'œil sec.

2. Composition ophtalmique comprenant de l'acide hyaluronique et de l'arabinogalactane en quantités synergiques selon la revendication 1, pour son utilisation dans le traitement du syndrome de l'œil sec accompagné des phénomènes inflammatoires associés à l'action du système enzymatique xanthine déshydrogénase/xanthine oxydase, qui sont réduits en raison de l'action combinée et synergique des deux principes actifs acide hyaluronique et arabinogalactane.

3. Composition ophtalmique selon la revendication 1 ou 2, comprenant en outre du panthénol, du tréhalose et de la vitamine E-D-α-polyéthylène glycol (TGPS), pour son utilisation dans le traitement du syndrome de l'œil sec, accompagné des phénomènes inflammatoires associés à l'action du système enzymatique xanthine déshydrogénase/xanthine oxydase.

4. Composition ophtalmique pour son utilisation selon l'une quelconque des revendications 1 à 3, **caractérisée en ce que** la quantité d'acide hyaluronique se trouve dans la plage de 0,15 % à 0,4 % en poids, sur le poids total.

5. Composition ophtalmique pour son utilisation selon l'une quelconque des revendications 1 à 3, **caractérisée en ce que** la quantité d'arabinogalactane se trouve dans la plage de 0,40 % à 1,00 % en poids, sur le poids total.

6. Composition ophtalmique pour son utilisation selon la revendication 3, **caractérisée en ce que** la quantité de panthénol se trouve dans la plage de 0,30 % à 0,60 % en poids, sur le poids total.

7. Composition ophtalmique pour son utilisation selon la revendication 3, **caractérisée en ce que** la quantité de tréhalose se trouve dans la plage de 3,00 % à 4,00 % en poids, sur le poids total.

8. Composition ophtalmique pour son utilisation selon la revendication 3, **caractérisée en ce que** la quantité de vitamine E-D-α-polyéthylène glycol (TGPS) est égale à 0,030 % en poids, sur le poids total.

9. Composition ophtalmique pour son utilisation selon la revendication 3, **caractérisée en ce qu'**elle peut être filtrée et stérilisée à 0,2 micron.

10. Composition ophtalmique pour son utilisation selon la revendication 1 ou 3, comprenant en outre un ou plusieurs agents thérapeutiques supplémentaires, sélectionnés dans le groupe comprenant des facteurs de croissance, des agents anti-inflammatoires, des vitamines, de la fibronectine et du collagène, des acides aminés et/ou des substances pharmacologiquement acceptables pour le traitement du syndrome de l'œil sec accompagné des phénomènes inflammatoires associés à l'action du système enzymatique xanthine déshydrogénase/xanthine oxydase.

11. Utilisation d'une association d'acide hyaluronique et d'arabinogalactane, en quantités synergiques, pour la préparation, après la stérilisation par filtration, d'un produit ophtalmique pour une utilisation topique sous la forme de gouttelettes oculaires pour le traitement du syndrome de l'œil sec.

12. Utilisation d'une composition comprenant une association d'acide hyaluronique et d'arabinogalactane, comprenant en outre du panthénol, du tréhalose et de la vitamine E-D-α-polyéthylène glycol (TGPS), pour la production de gouttelettes oculaires pour le traitement du syndrome de l'œil sec, après stérilisation par filtration.

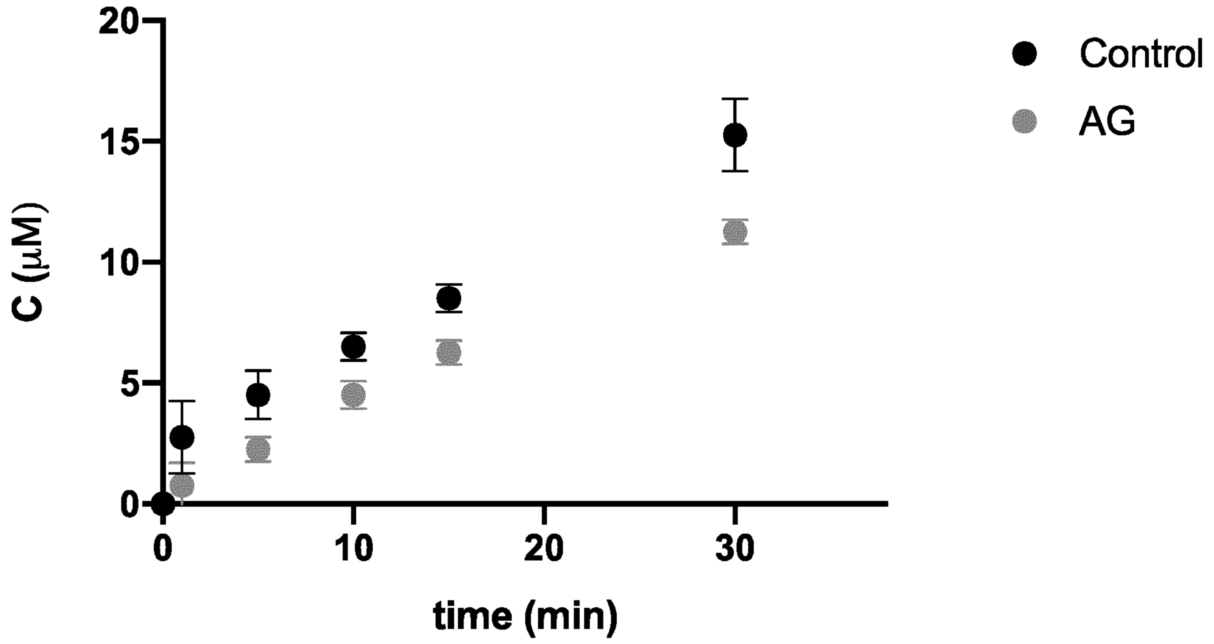
20
15
10
5
0
C (μM)
0
10
20
30
time (min)
Control
AG

FIG. 1a

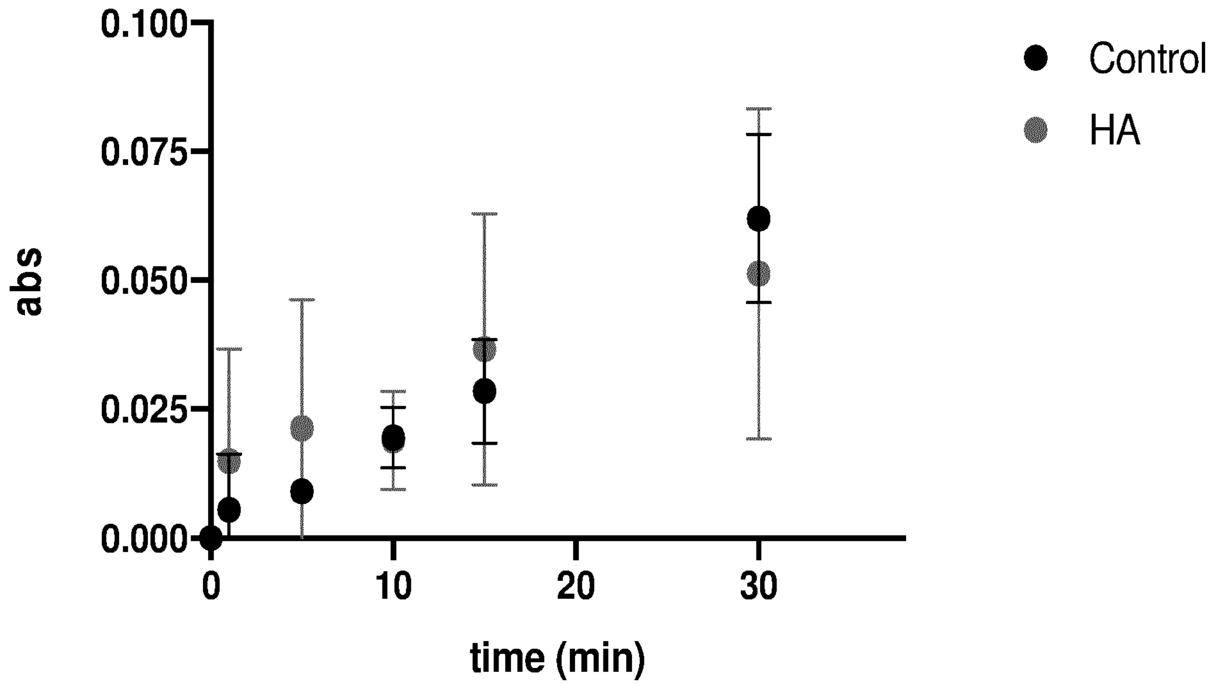
0.100
0.075
0.050
0.025
0.000
abs
0
10
20
30
time (min)
Control
HA

FIG.1b

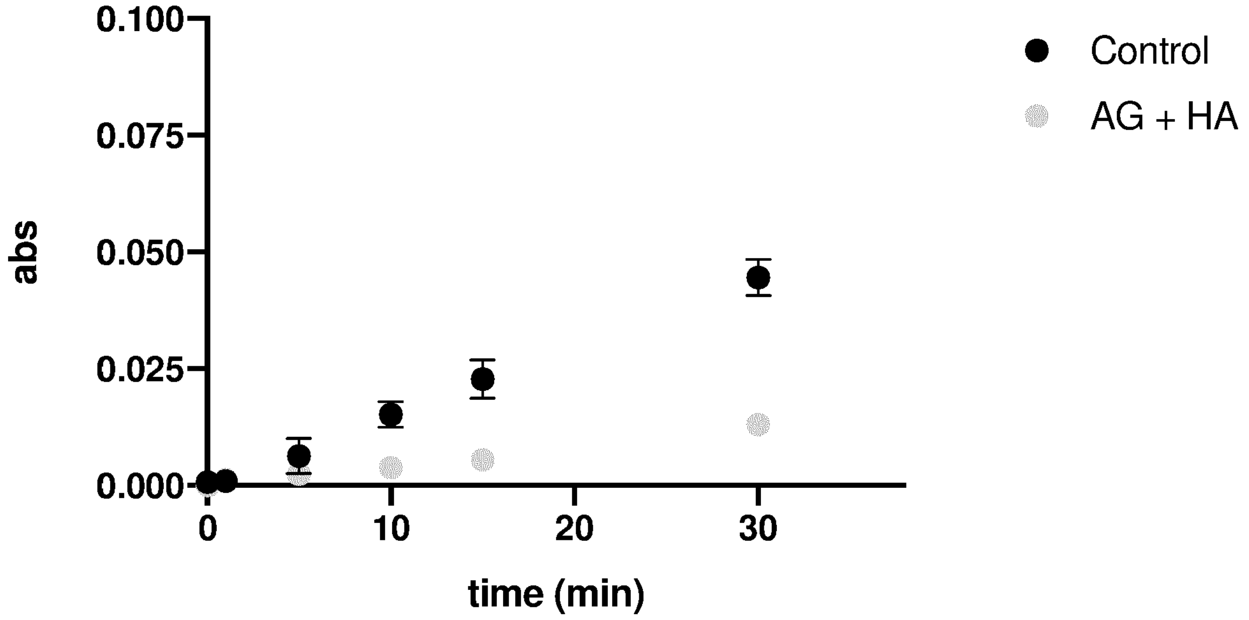
0.100
0.075
0.050
0.025
0.000
abs
0
10
20
30
time (min)
Control
AG + HA

FIG.1c

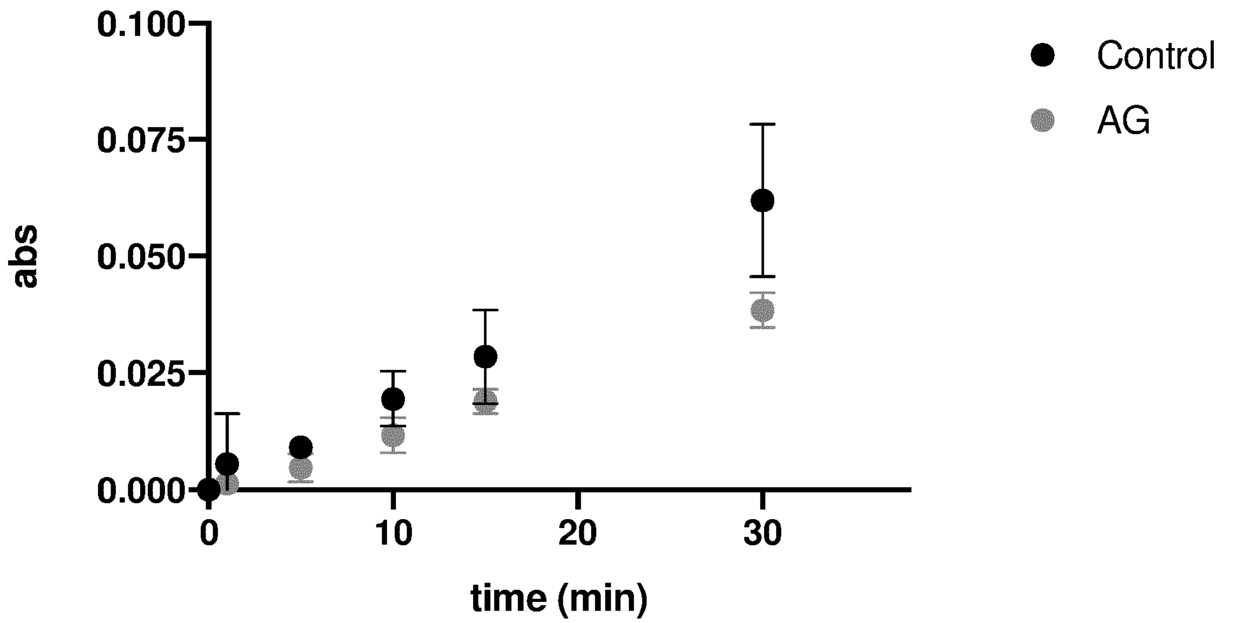
0.100
0.075
0.050
0.025
0.000
abs
0
10
20
30
time (min)
Control
AG

FIG.2a

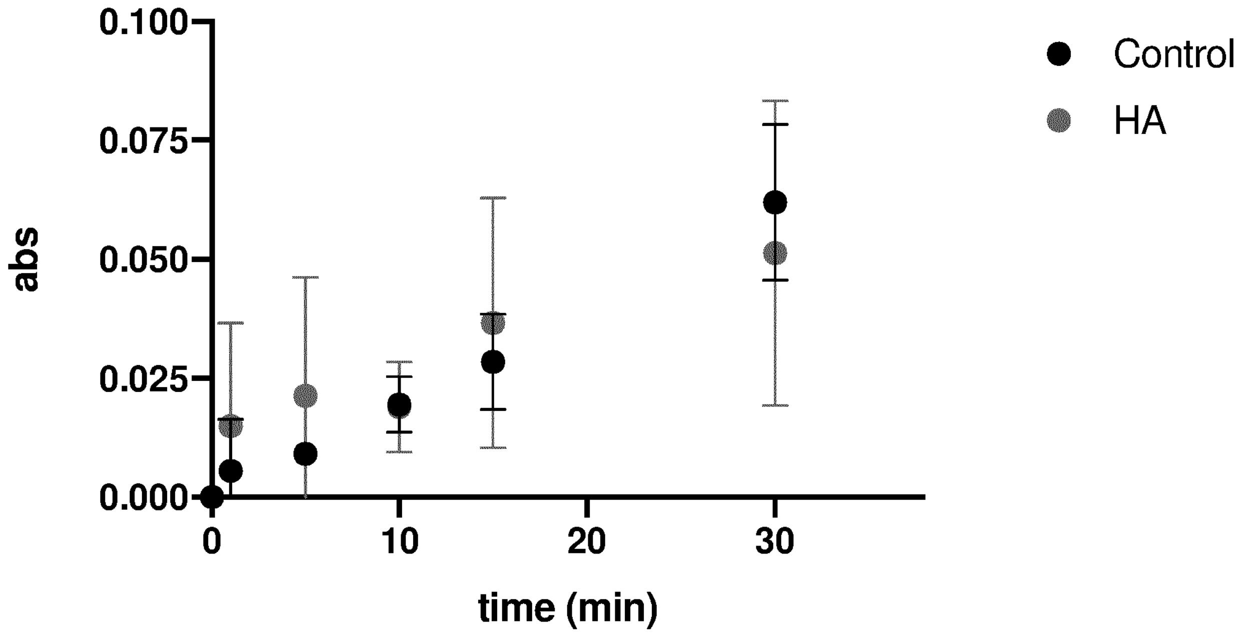
0.100
0.075
0.050
0.025
0.000
abs
0
10
20
30
time (min)
Control
HA

FIG.2b

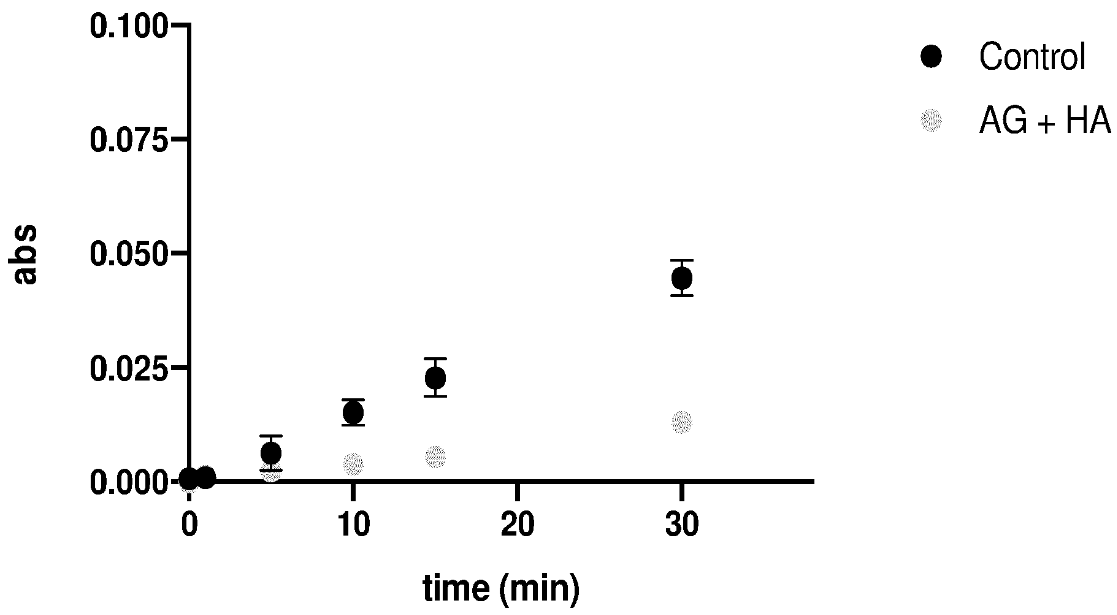
0.100
0.075
0.050
0.025
0.000
abs
0
10
20
30
time (min)
Control
AG + HA

FIG.2c.

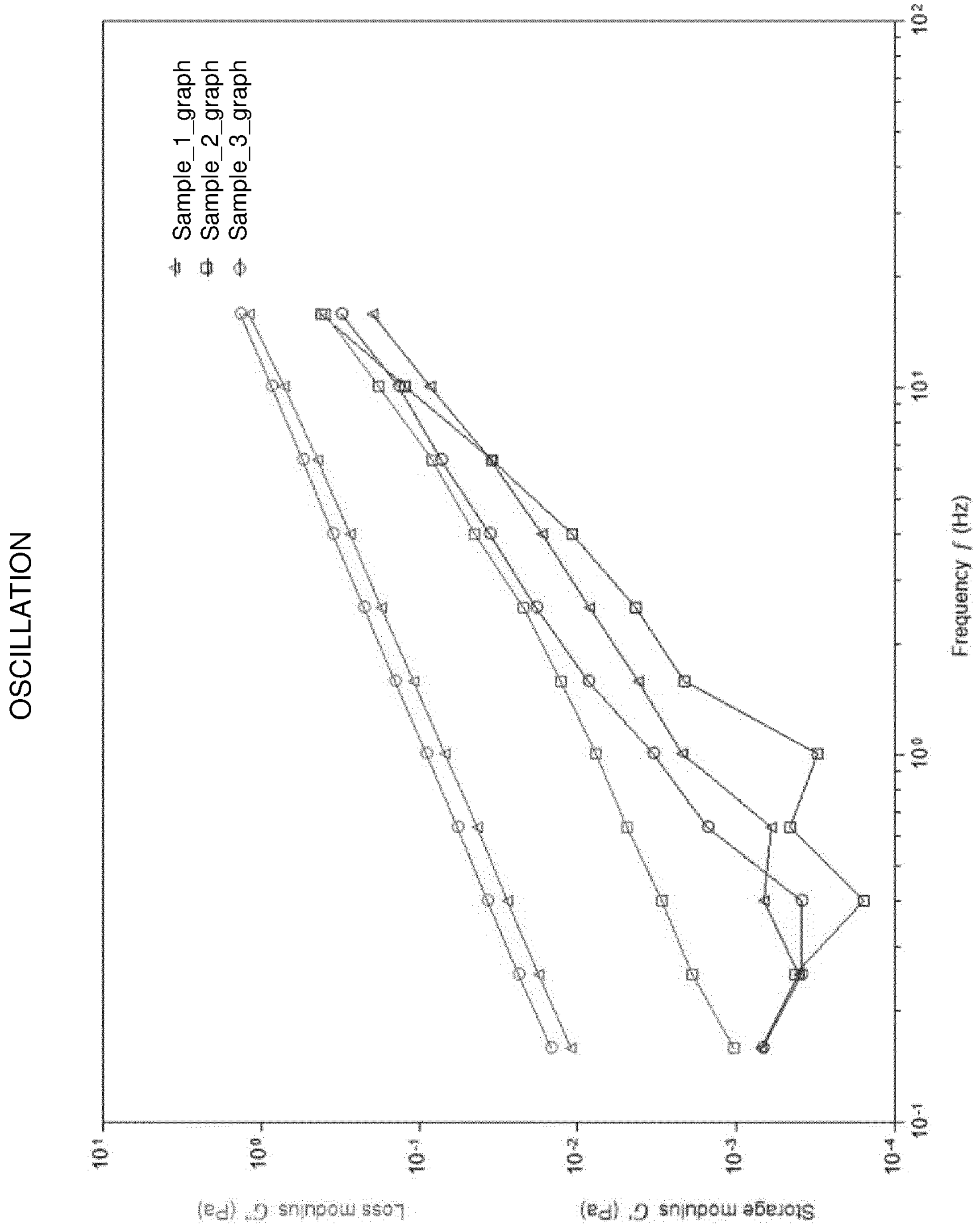
OSCILLATION
Sample_1_graph
Sample_2_graph
Sample_3_graph
Loss modulus G'' (Pa)
Storage modulus G' (Pa)
Frequency f (Hz)
10^1
10^0
10^-1
10^-2
10^-3
10^-4
10^-1
10^0
10^1
10^2

FIG.3

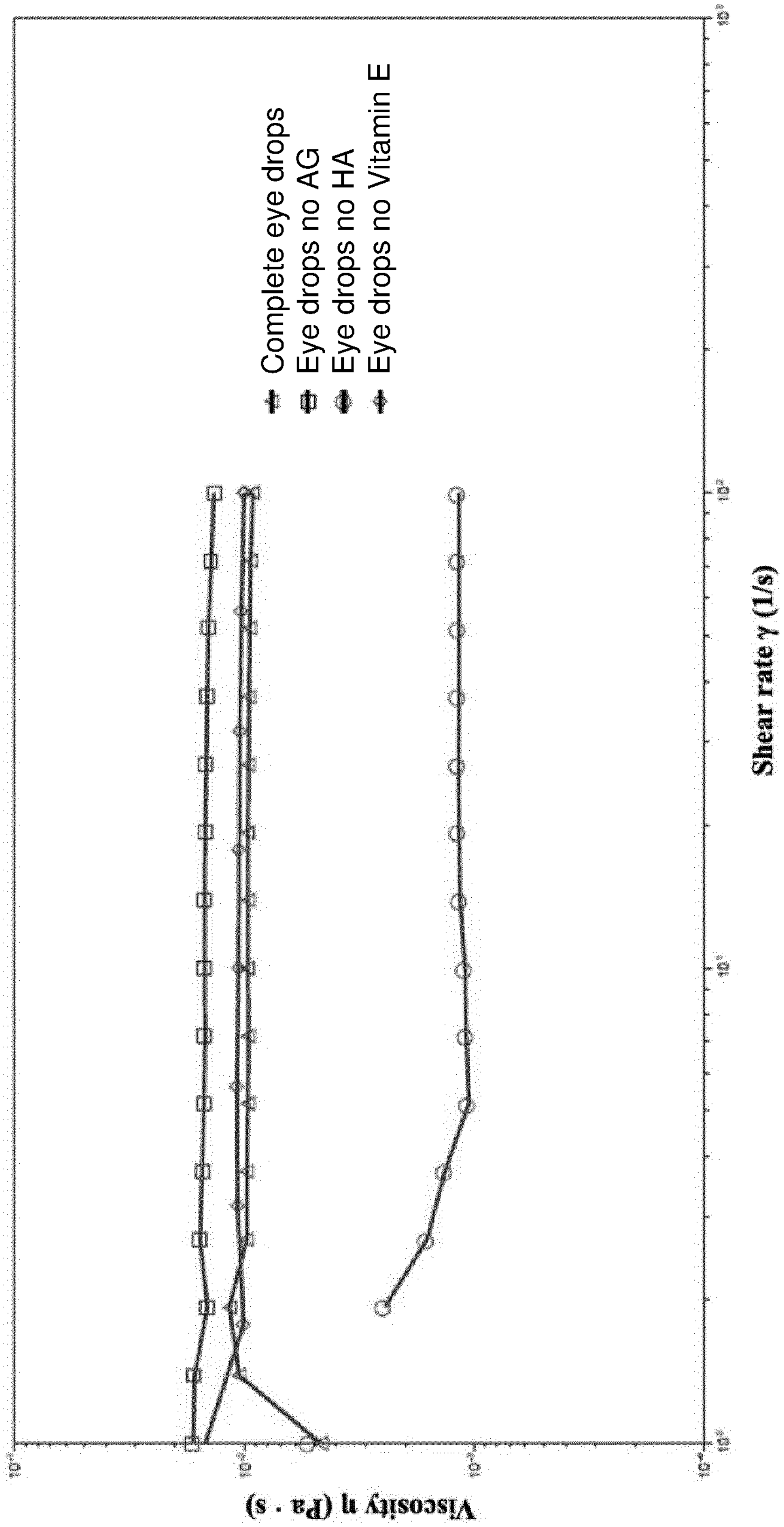
Complete eye drops
Eye drops no AG
Eye drops no HA
Eye drops no Vitamin E
Viscosity η (Pa · s)
Shear rate γ (1/s)

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009018060 A
- EP 1912707 A


### Non-patent literature cited in the description


- **BURGALASSI et al.** *Curr Eye Res.,* 2011, vol. 36 (1), 21-8
- **BAUDOIN et al.** *Acta Ophthalmol,* 2018, vol. 96 (2), 111-119
- **VAZIRANI.** *Indian J Ophthalmol,* 2018, vol. 66 (1), 44
- **YAGCI ; GURDAL.** *Int Ophthalmol,* 2014, vol. 34 (6), 1291-1301
- **CEJKOVA et al.** *Histol Histopathol,* 2007, vol. 22 (9), 997-1003
- **KUPPUSAMY ; ZWEIER.** *The Journal of biological chemistry,* 1989, vol. 264, 9880-9884
- **SERY ; PETRILLO.** *Journal of current eye research,* 1984, vol. 1, 243-252
- **PUSTIVRH et al.** *Reproduction, fertility and development,* 2000, vol. 12 (6), 269-275
- **OWEN ; JOHNS.** *J Ethnopharmacol.,* 1999, vol. 64 (2), 149-160
- **ANUBHA KHARE et al.** *Mucoadhesive Polymers for Enhancing Retention in Ocular Drug Delivery: A Critical Review,* 2014
- **ISLAM MAHMOUD HAMDI.** Effect of D-Panthenol on corneal epithelial healing after surface laser ablation. *Journal of Ophthalmology,* 2018
- **TANGE T.** *J Magn Resin,* 2007, vol. 184 (2), 222-227
- **LI et al.** *Molecular Vison,* 2012, vol. 16, 317-329
- **HISTOL HISTOPATHOL.** *Xanthine oxidoreductase and xanthine oxidase in human cornea,* 2002, vol. 17, 755-760
- **BURGALASSI S ; NICOSIA N ; MONTI D ; FALCONE G ; BOLDRINI E ; FABIANI O ; LENZI C ; PIRONE A ; CHETONI P.** Larch arabinogalactan for dry eye protection and treatment of corneal lesions: investigations in rabbits. *J Ocul Pharmacol Ther.,* December 2007, vol. 23 (6), 541-50
- **ROLANDO M1 ; VALENTE C.** Establishing the tolerability and performance of tamarind seed polysaccharide (TSP) in treating dry eye syndrome: results of a clinical study. *Ophthalmol.,* 29 March 2007, vol. 7, 5
- **ISLAM MAHMOUD HAMDI.** Effect of D-Panthenol on Corneal Epithelial Healing after Surface Laser Ablation. *Journal of Ophthalmology,* vol. 2018, 6
- **MING TANG ; A ALAN ; J. WARING,B ; MEI HONGA.** Trehalose-Protected Lipid Membranes for Determining Membrane Protein Structure and Insertion. *J Magn Reson.,* February 2007, vol. 184 (2), 222-227
- **JINYANG LI ; CHRISTOPHE ROUBEIX ; YU WANG ; SHUAI SHI ; GUOTING LIU ; CHRISTOPHE BAUDOUIN ; WEI CHEN.** Therapeutic efficacy of trehalose eye drops for treatment of murine dry eye induced by an intelligently controlled environmental system. *Molecular Vison,* 2012, vol. 16, 317-329
- **CHRISTOPHE BAUDOUIN ; MURAT IRKEÇ ; ELISABETH M. MESSMER ; JOSÉ M. BENITEZ DEL CASTILLO ; STEFANO BONINI.** Clinical impact of inflammation in dry eye disease: proceedings of the ODISSEY group meeting. *Acta Ophthalmol,* March 2018, vol. 96 (2), 111-119
- **JAYESH VAZIRANI.** Ocular surface inflammation in dry eye disease: What we know and what we do not. *Indian J Ophthalmol,* January 2018, vol. 66 (1), 44
- **YAGCI A1 ; GURDAL C.** The role and treatment of inflammation in dry eye disease. *Int Ophthalmol,* 26 July 2014, vol. 34 (6), 1291-301
- **CEJKOVÁ J1 ; ARDAN T ; JIRSOVÁ K ; JECHOVÁ G ; MALEC J, SIMONOVÁ Z ; CEJKA C ; FILIPEC M ; DOTRELOVÁ D ; BRUNOVÁ B.** The role of conjunctival epithelial cell xanthine oxidoreductase/xanthine oxidase in oxidative reactions on the ocular surface of dry eye patients with Sjögren's syndrome. *Histol Histopathol,* September 2007, vol. 22 (9), 997-1003
- **P KUPPUSAMY ; J L ZWEIER.** Characterization of free radical generation by xanthine oxidase. Evidence for hydroxyl radical generation. *The Journal of Biological Chemistry,* 15 June 1989, vol. 264, 9880-9884

- **THEODORE W. ; RAYMOND PETRILLO ; THOMAS JEFFERSON.** nSuperoxide anion radical as an indirect mediator in ocular inflammatory disease. *Journal Current Eye Research,* 1984, vol. 3 (1), 243-252
- **CAROLINA PUSTOVRH ; ALICIA JAWERBAUM ; DEBORA SINNER ; MARIO PESARESI ; MARIO BAIER ; PAULA MICONE ; MARTHA GIMENO ; ELIDA T. GONZALEZ.** Membrane-type matrix metalloproteinase-9 activity in placental tissue from patients with pre-existing and gestational diabetes mellitus. *Reproduction, Fertility and Development,* 2000, vol. 12 (6), 269-275
- **OWEN PL1 ; JOHNS T.** Xanthine oxidase inhibitory activity of northeastern North American plant remedies used for gout. *J Ethnopharmacol.,* February 1999, vol. 64 (2), 149-60
- **MASSEY,V.** Handbook of Methods for oxygen radical Research Third Edition. 1987, 213-2015
- **IRWIN FRIDOVICH.** *Biock.Biophys.Acta,* 1959, vol. 34 (255), 17
- *Biochem Pharmacol.,* 15 May 1990, vol. 39 (10), 1597-601